# EUROPÄISCHE PATENTSCHRIFT

(11) **EP 0 845 013 B1**
(45) Veröffentlichungstag und Bekanntmachung des Hinweises auf die Patenterteilung: **24.10.2001**
(21) Anmeldenummer: 96928472.8
(22) Anmeldetag: 16.08.1996
(51) Int. Cl.: C08K 5/3435, C08L 23/10, C08L 53/00

(54) **VERWENDUNG VON PIPERIDINVERBINDUNGEN**
USE OF PIPERIDINE COMPOUNDS
UTILISATION DE COMPOSES DE PIPERIDINE

(30) Priorität: 18.08.1995 DE 19530468
(43) Veröffentlichungstag der Anmeldung: 03.06.1998
(73) Patentinhaber: Clariant Finance (BVI) Limited, Road Town, Tortola (VG); SANDOZ-PATENT-GMBH, 79539 Lörrach (DE)
(72) Erfinder: AVAR, Lajos, CH-4105 Biel-Benken (CH); LIGNER, Gilbert, F-68920 Wintzenheim (FR); THUERMER, Andreas, F-68300 Saint-Louis (FR)
(74) Vertreter: D'haemer, Jan Constant
(86) Internationale Anmeldenummer: EP9603622
(87) Internationale Veröffentlichungsnummer: WO9707159

(56) Entgegenhaltungen:
- EP-A- 0 389 427
- EP-A- 0 437 096
- DE-A- 3 412 227
- GB-A- 2 269 819
- RESEARCH DISCLOSURE, Bd. 389, Nr. 010, 10.September 1996, EMSWORTH, GB, XP002020798 ANONYMOUS: "'SANDUVOR PR-31'(RTM) as UV light stabiliser for polymeric materials - which acts as both UV absorber and hindered amine light stabiliser, combined with antioxidant properties" & DATABASE WPI Week 9644 Derwent Publications Ltd., London, GB; AN 96-441170 & CHEMICAL ABSTRACTS, vol. 125, no. 20, 1996 Columbus, Ohio, US; abstract no. 249396,

## Beschreibung

Die Erfindung betrifft den Gegenstand der Patentansprüche.

Die Lichtstabilisierung von Formteilen aus EPDM-modifiziertem Polypropylen im Automobilbau, insbesondere von Autostossfängern, ist aufgrund des heterogenen Aufbaus der Polymermatrix und ihrer nur partiellen Kristallinität eine besonders anspruchsvolle Aufgabe, da (a) das Ausblühen des Lichtschutzstabilisators an die Oberfläche verhindert werden und (b) die Lichtschutzstabilisation auf hohem Niveau und guter Permanenz über eine lange Zeitperiode hinweg gesichert sein muss.

Aufgabe der vorliegenden Erfindung war daher, EPDM-modifiziertes Polypropylen, das insbesondere im Automobilbau Verwendung findet, gegen Lichteinfluss zu stabilisieren.

Diese Aufgabe wird durch die vorliegende Erfindung gelöst und ferner der Stand der Technik deutlich übertroffen. Es wurde nämlich gefunden, dass das Ausblühen des Stabilisators verhindert werden kann, wenn man diesen chemisch an das EPDM-modifizierte Polypropylen bindet. Die chemische Bindung zum EPDM-modifizierten Polypropylen erfolgt durch Belichten des den erfindungsgemässen Stabilisator der untenstehenden Formel I enthaltenden EPDM-modifizierten Polypropylens - mit energiereichem Licht, wobei die Bindung zur Polymeroberfläche bzw. innerhalb der Polymermatrix in oberflächennahen Schichten ausgebildet wird. Die Lichtschutzstabilisierung auf hohem Niveau wird dadurch gewährleistet, dass die im Innern eines Formkörpers, der aus mit erfindungsgemässen Stabilisatorverbindungen enthaltenden EPDM-modifizierten Polypropylen hergestellt ist, befindlichen Stabilisatorverbindungen langsam zur Oberfläche diffundieren und dort durch die im Gebrauch erfolgende Belichtung chemisch an das EPDM-modifizierte Polypropylen gebunden werden. So werden ggf. zerstörte Stabilisatorverbindungen an der Oberfläche immer wieder ersetzt und die hohe Permanenz der UV-Stabilisierung aufrecht erhalten.

Gegenstand der Erfindung ist somit die Verwendung von Piperidinverbindungen der Formel I 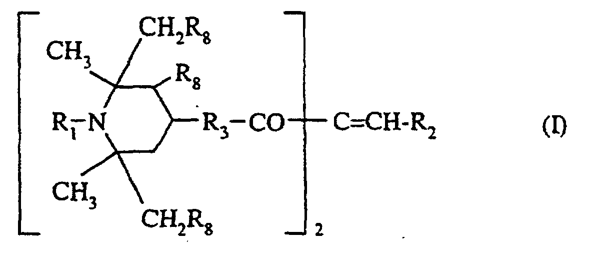 worin
- R₁: Wasserstoff, niedermolekulares Alkyl, Acyl
- R₂: einen gegebenenfalls Substituenten tragenden ein- oder zweikernigen Rest aromatischen Charakters,
- R₃: Sauerstoff, -NH- oder -N(C₁₋₄-Alkyl)- und
- alle R₈: unabhängig voneinander Wasserstoff oder Methyl
bedeuten,
zur Herstellung von lichtstabilisiertem, EPDM-modifiziertem Polypropylen, das insbesondere im Automobilbau, z.B. für Schockabsorber, Abdeckprofile und dergleichen eingesetzt wird.

Unter niedrigmolekularem Alkyl sind vorzugsweise Reste mit 1-8, insbesondere 1 oder 2 Kohlenstoffatomen zu verstehen; als "Acyl" kommen vorzugsweise die Reste der Ameisen-, Essig- oder Propionsäure in Betracht.

Alle R₁ sind vorzugsweise Wasserstoff oder Methyl, vor allem Methyl.

Unter ein- oder zweikemigen Resten R₂ aromatischen Charakters kommen z. B. Benzol-, Naphthalin und Stickstoff und/oder Schwefel enthaltende Fünf- oder Sechserringe, die gegebenenfalls an einen Benzolring anelliert sind, in Betracht, die z. B. sterisch gehindertes Hydroxyl als Substituenten tragen (3,5-ditert.-Butyl-4-hydroxyphenyl), oder ein Thienylrest. Bevorzugt sind aromatische Sechsringe. Als Substituenten an diesen Ringen können z. B. Hydroxyl, niedermolekulares Alkyl oder Alkoxy, vorzugsweise Methyl, tert.- Butyl, Methoxy, Aethoxy, Hydroxyl und eine oder zwei weitere Gruppen der Formel 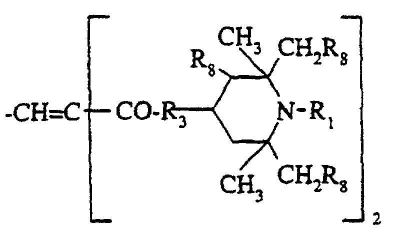 genannt werden.
Alle R₈ bedeuten vorzugsweise Wasserstoff.

Als zusätzliche Stabilisatoren können für das EPDM-modifizierte Polypropylen u.a. Antioxidantien, z. B. sterisch gehinderte Phenole, sekundäre aromatische Amine oder Thioäther (beschrieben z. B. in "Plastics Additives", Gächter und Müller, 1985, S. 8-12), weitere HALS-Verbindungen, Antistatika, entflammungsverhindernde Mittel, Weichmacher, Nukleiermittel, Metall-Desaktivatoren, Biocide und dergleichen eingesetzt werden.

Weiterer Gegenstand der Erfindung ist die Kombination aus Piperidinverbindungen der Formel I mit bekannten UV-Absorbem (2-(2'-Hydroxyphenyl)-benztirazolverbindungen, 2-Hydroxy-benztriazolverbindungen, 1,3-Bis-(2'-hydroxybenzoyl)-benzolsalicylate, Zimtsäurederivate, Triazinderivate und Oxalsäureanilide). Es wurde gefunden, dass durch Mischungen von Verbindungen der Formel I und UV-Absorbern im Verhältnis 10 : 1 bis 1 : 10, bevorzugt 4 : 1 bis 1 : 4, je nach Dicke des zu stabilisierenden Gegenstands, das Gleichgewicht von Diffusionsgeschwindigkeit und photochemischer Bindung von Stabilisatoren der Formel I an die Polymermatrix beeinflusst werden und somit die Lichtschutzwirkung verbessert werden kann.

Die folgenden Verbindungen der Formeln Ia und Ib sind besonders gut für die Lichtstabilisierung von EPDM-modifiziertem Polypropylen geeignet: 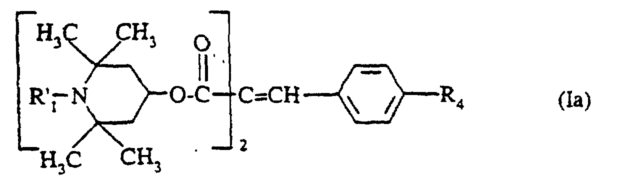 worin
- R₄ =: H, OCH₃
- R'₁ =: H, CH₃,
bedeuten, und 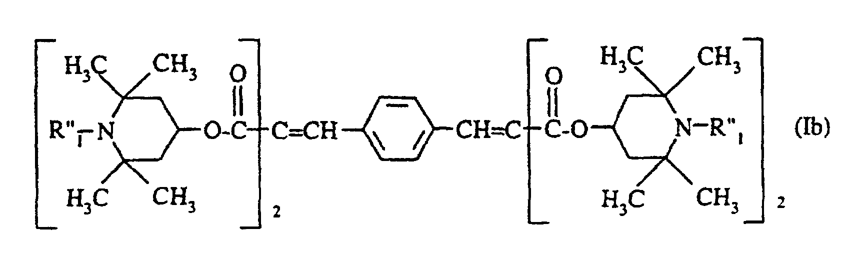 worin
- R" ₁=: H, CH₃
bedeutet.

Die chemische Bindung zum EPDM-modifizierten Polypropylen erfolgt während des Gebrauchs und damit bei Licht-Exposition des die erfindungsgemässe Piperidinverbindung enthaltenden, EPDM-modifizierten Polypropylens mit energiereichem sichtbarem oder nahem UV-Licht, wobei die hierzu erforderliche Belichtungsdauer im allgemeinen zwischen etwa 100 und 400 Stunden beträgt. Die Belichtung kann mit einer Xenon-Lampe oder mit Sonnenlicht erfolgen. Hierbei brechen die Doppelbindungen in der Verbindung der Formel I auf, und es erfolgt eine chemische Bindung an entsprechend reaktionsfähigen Kohlenstoffatomen des EPDM-modifizierten Polypropylens.

Bei voluminösen Formkörpern wie Autostossstangen, werden die im Innern der Formkörper befindlichen HALS-Verbindungen (das sind die als Stabilisator wirkenden Piperidinverbindungen bzw. -Reste) langsam gegen die Oberflächen diffundieren und dort, durch die im Gebrauch erfolgende Belichtungm, chemisch an das EPDM-modifizierte Polypropylen gebunden. So werden gegebenenfalls zerstörte HALS-Stabilisatoren an der Oberfläche immer wieder ersetzt und die Stabilisierung auf wesentlich längere Zeit ausgedehnt.

Im allgemeinen setzt man 0,05 bis 2,5%, bevorzugt 0,1 bis 0,6% einer oder mehrerer Verbindungen der Formel I, bezogen auf das Gewicht des zu stabilisierenden EPDM-modifizierten Polypropylens eingesetzt, je nach angestrebtm Grad der Stabilisierung bzw. Gebrauchsdauer (1, 2 oder 10 Jahre) oder geographischem Einsatzgebiet des fertigen Polymerartikels.

Polymerartikel, die gemäss vorliegender Erfindung gegen den schädigenden Einfluss von UV-Licht stabilisiert wurden, zeichnen sich gegenüber dem Stand der Technik dadurch aus, dass bereits nach kurzer Gebrauchszeit und damit Licht-Exposition die Stabilisatoren (Verbindungen der Formel I) auf physikalischem Weg, selbst durch Eluieren mit Lösungsmitteln, nicht mehr aus dem EPDM-modifiziertem Polypropylen entfernt werden können. Bei üblichem Gebrauch der Polymerartikel ist das Auswaschen, d.h. der Verlust von Stabilisatoren, auf klimatische und/oder Umgebungseinflüsse (z. B. Kontakt mit Reinigungsflüssigkeiten, Ölen, Fetten oder sonstigen Chemikalien) zurückzuführen. Auch unter diesen Bedingungen zeigen die erfindungsgemäss UV-stabilisierten Polymere eine hohe Resistenz. Im allgemeinen wird basisstabilisiertes, EPDM-modifiziertes Polypropylen als Grundmaterial verwendet und in einem zweiten Verarbeitungsschritt erfindungsgemäss UV-stabilisiert. Die Basisstabilisierung erfolgt im allgemeinen, wie bekannt, mit 0.05-0.2% eines phenolischen Antioxidans und 0.05-0.5% Calciumstearat, bezogen auf das Gewicht des EPDM-modifizierten Polypropylens.

Die Herstellung von Verbindungen der Formel I [(desgleichen die der Formel (Ia) und (Ib)] ist bekannt und werden durch Kondensation eines Mols der Verbindung der Formel II

R₂ - CH = C = (COOH)₂ (II)

oder eines funktionellen Säurederivats, z. B. eines niedermolekularen Esters oder eines Säurehalogenids, mit 2 Mol einer Verbindung der Formel III

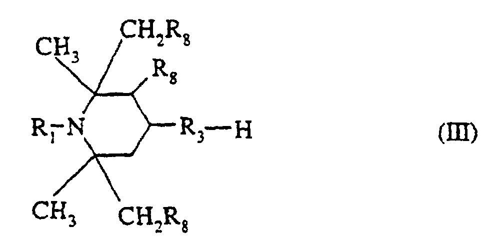

In den folgenden Beispielen bedeuten die Teile Gewichtsteile und die Prozente Gewichtsprozente. Die Volumenteile entsprechen dem Volumen gleicher Gewichtsteile Wasser. Die Temperaturen sind in Celsiusgraden angegeben.

### I. Herstellung von Verbindungen der Formel I

### Beispiel 1

84 Teile para-Methoxy-benzalmalonsäuredimethylester werden mit 154 Teilen 1,2,2,6,6-Pentamethyl-4-hydroxypiperidin in 80 Teilen Xylol verteilt, auf 80° erhitzt, mit 0,5 Volumenteilen Tetraisopropyl-orthotitanat versetzt und, unter Abdestillieren des entstehenden Methanols, 22 Stunden bei etwa 140° gerührt. Die Reaktionslösung wird sodann auf 80° gekühlt, dreimal mit 150 Teilen Wasser gewaschen, die organische Phase abgetrennt, das Lösungsmittel abdestilliert und der Rückstand aus Aethanol umkristallisiert. Das erhaltene Produkt der Formel 1 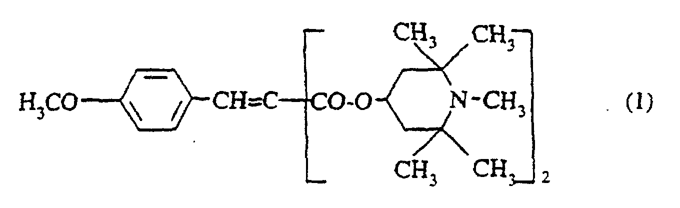 schmilzt bei 120- 125°.

### II. Herstellung von basisstabilisiertem EPDM-modifizierten Polypropylen

### Beispiel 2

Aus einem EPDM-modifizierten Polypropylen, bestehend aus 55-70 % Ethylen, 28-43 % Propylen und 1,8-3,3 % Hexadien, Dicyclopentadien oder Ethylvinyliden (vernetzt durch Schwefel-Vulkanisation oder radikalisch durch Peroxidzusatz), das einen Schmelzindex von 10 dg/min (230°/2,16 kg) besitzt, und den drei folgenden Basisstabilisatoren wurde eine rieselfähige Trockenmischung hergestellt, die anschliessend in einem Einschneckenextruder (1 : d = 20, Kompressionsverhältnis 1 : 3, Schneckendurchmesser = 20 mm, 50 Umdrehungen pro Minute) bei 230° verarbeitet wurde.

| Basisstabilisierung: | | |
|---|---|---|
| 800 | ppm Irganox® 1010 | (Ciba-Geigy), ein Antioxidans auf Basis eines sterisch gehinderten Phenols |
| 1000 | ppm Sandostab® P-EPQ | (Clariant), ein Verarbeitungsstabilisator auf Basis eines sterisch gehinderten Phosphonits |
| 800 | ppm Calciumstearat | |

### Beispiel 3

Aus einem EPDM-modifizierten Polypropylen (vernetzt durch Schwefel-Vulkanisation oder radikalisch durch Peroxidzusatz) mit einem Gehalt an EPDM-Kautschuk von 20-25%, das einen Schmelzindex von 6 dg/min (230°/2,16 kg) besitzt, und den zwei folgenden Basisstabilisatoren wurde eine rieselfähige Trockenmischung hergestellt, die in einem Einschneckenextruder (1 : d = 20, Kompressionsverhältnis 1 : 3, Schneckendurchmesser = 20 mm, 50 Umdrehungen pro Minute) bei 230° kompoundiert wurde.

| Basisstabilisierung: | | |
|---|---|---|
| 1500 | ppm Irganox® B-225 | (Ciba-Geigy), ein Antioxidans aus gleichen Teilen Irganox 1010 (s.o.) und Irgafos® 168, einem Phosphit-Verarbeitungsstabilisator |
| 800 | ppm Calciumstearat | |

### Beispiel 4

Ein schlagzäh-modifitiertes Polypropylen-Blockcopolymer mit einem Ethylengehalt von 4% und einem Schmelzindex von 5,0 dg/min (230°/2,16 kg) wurde mit den drei folgenden Basisstabilisatoren stabilisiert.

| Basisstabilisierung: | | |
|---|---|---|
| 1000 | ppm Irganox® 1010 | (Ciba-Geigy), ein Antioxidans auf Basis eines sterisch gehinderten Phenols |
| 500 | ppm Sandostab® P-EPQ | (Clariant), ein Verarbeitungsstabilisator auf Basis eines sterisch gehinderten Phosphonits |
| 1000 | ppm Calciumstearat | |

### III. Erfindungsgemäss stabilisiertes EPDM-modifiziertes Polypropylen

Folgende Messungen wurden einerseits an erfindungsgemäss und andererseits dem Stand der Technik gemäss stabilisiertem Polypropylen ausgeführt:
a) Künstliche Bewitterung nach DIN 52231-A:
   Beurteilung erfolgt visuell hinsichtlich -oberflächenbeschläge oder -Risse, Glanz, Elastizität, Biegsamkeit und Verfärbung.
b) Langzeit-Wärmealterungsversuch nach DIN 53383
   Fehlerkriterium ist der Biegetest von spritzgegossenen Prüftafeln nach Lagerung in einem Umluftofen, d.h. der Bruch der Probe bei Beurteilung.
c) Kurzbewitterung nach DIN 53231-A
   Das Versagen der Proben wird anhand von mechanischen Messungen beurteilt:
   - Dünne Probekörper: Zugfestigkeit nach DIN 53455
   - Dicke Prüftafeln: Schlagzähigkeit nach DIN 53453

Spritzgegossene Prüftafeln und Prüfstabe wurden mit einer Spritzgussmaschine vom Typ Ahrburg IM bei einer Temperatur von 220 - 230°, 80 Umdrehungen pro Minute, einem Spritzdruck von 100 bar und einer Schliesskraft von 12 kN hergestellt.

### Beispiel Aa und Vergleichsbeispiel Ab

Spritzgegossene Prüftafeln von 2 mm Dicke, hergestellt aus dem basisstabilisierten EPDM-modifizierten Polypropylen von Beispiel 2, dem zusätzlich folgende Verbindungen als Lichtschutzmittel zugesetzt wurden, wurden künstlich bewittert.

| **Beispiel** | **Lichtschutzstabilisator** | **Versagen nach** |
|---|---|---|
| **Aa** | 4500 ppm von Verbindung | 3500 h |
| | 1 von Beispiel 1 | |
| **Ab** | 5000 ppm von | 2500 h |
| | Sanduvor® 3944* | |

| | | |
|---|---|---|
| ** Sanduvor*® *3944 ist ein Lichtschutzstabilisator mit einem mittieren Molekulargewicht von* > *2500 und einem Schmelzbereich von 100- 135° der Fa*. *Clartant der Formel* | | |

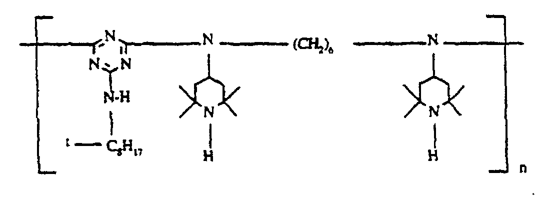

Der vorliegende Vergleich zeigt eine deutlich verbesserte Standzeit der erfindungsgemäss stabilisierten Probe Aa (trotz 10% geringerer Einsatzkonzentration) gegenüber mit handelsüblicher HALS-Verbindung ausgerüsteten Material Ab gemäss dem gegenwärtigen Stand der Technik.

### Beispiel Ba und Vergleichsbeispiele Bb - Bd

Die Langzeitwärmealterung wurde an spritzgegossenen Prüftafeln von 3 mm Dicke, hergestellt aus basisstabilisiertem, EPDM-modifiziertem Polypropylen von Beispiel 3, dem folgende Lichtschutzmittel zugesetzt wurden, gemessen:

| **Beispiel** | **Lichtschutzstabilisator** | **Versagen nach** |
|---|---|---|
| **Ba** | 4500 ppm Verbindung von Beispiel 1 | 30 Tagen |
| **Bb** | 5000 ppm Sanduvor® 3944 | 14 Tagen |
| **Bc** | 3000 ppm Tinuvin® 770* | 14 Tagen |
| | 2000 ppm Sanduvor® 3944 | |
| **Bd** | 6000 ppm Tinuvin® 770 | 7 Tagen |

| | | |
|---|---|---|
| ** Tinuvin*® *770 ist ein Lichtschuizmittel der Fa*. *Ciba-Getgv der Formel* | | |

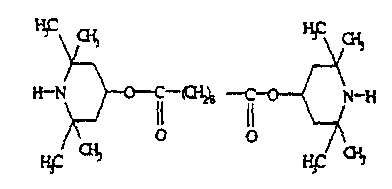

### Beispiele Da bis Dc und Vergleichsbeispiele Dd bis De

Ein Kurzbewitterungstest erfolgte an spritzgegossenen Prüfkörpern (1 mm Schulterstäbe, bzw. 3 mm Prüftafeln), hergestellt aus dem basisstabilisierten, schlagzähen Polypropylen-Blockcopolymer von Beispiel 4, dem folgende Lichtschutzmittel zugegeben wurden:

| Beispiel | Lichtschutzmittel HALS | UV-Absorber UVA | Zugfestigkeit (in % des ursprünglichen Wertes) | Schlagzähigkeit |
|---|---|---|---|---|
| **Da** | 2000 ppm | --- | 73% nach 3500 h | 88% |
| | Verbindung 1 | | | |
| **Db** | 1330 ppm | 1330 ppm | 96% nach 3500 h | 90% |
| | Verbindung 1 | Sanduvor® 3035* | | |
| **Dc** | 600 ppm | 2260 ppm | 95% nach 3500 h | 98% |
| | Verbindung 1 | Sanduvor® 3035 | | |
| **Dd** | 2000 ppm | --- | 50% nach 3000 h | 57% |
| | Sanduvor® 3944 | | | |
| **De** | --- | 4000 ppm | 50% nach 2900 h | 80% |
| | | Sanduvor® 3035 | | |

| | | | | |
|---|---|---|---|---|
| ** Sanduvor® 3035 ist ein UV-Absorber der Fa*. *Clartant mit der Formel* | | | | |

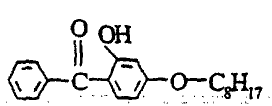

Bei den Konzentrationsbereichen, die für UV-Absorber in obigem Beispiel gewählt wurden, ist der bekannten Tatsache Rechnung getragen, dass UV-Absorber aufgrund ihrer deutlich geringeren Wirksamkeit als Lichtschutzmittel im Vergleich zu HALS-Verbindungen in etwa doppelter Konzentration im Polymer eingesetzt werden müssen, um - in dickeren Artikeln wie im vorliegenden Beispiel beschrieben - annähernd vergleichbare Lichtschutzeffekte zu erzielen (siehe Beispiel De gegenüber Da und Dd). Die Beipiele Db (50% HALS, 50% UVA) und Dc (20% HALS, 80% UVA) verdeutlichen die überragende Wirkung von Kombinationen erfindungsgemässer Lichtschutzmittel vom HALS-Typ mit UV-Absorbem, was in nur geringfügig veränderten mechanischen Eigenschaften nach der Belichtung, d.h. in der effizienten UV-Stabilisierung des Polymeren, klar zum Ausdruck kommt.

## Patentansprüche

1. Verwendung von Piperidinverbindungen der Formel (I) 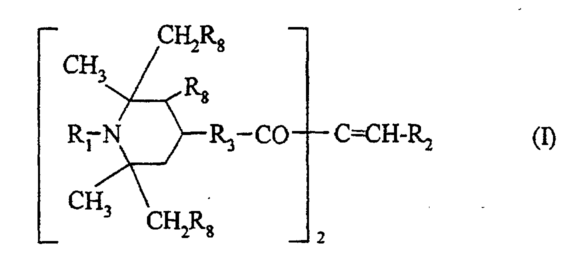 worin
R₁ Wasserstoff, Acyl oder niedermolekulares Alkyl,
R₂ einen gegebenenfalls Substituenten tragenden ein- oder zweikernigen Rest aromatischen Charakters,
R₃ Sauerstoff, -NH- oder -N(C₁₋₄-Alkyl)- und
alle R₈ unabhängig voneinander Wasserstoff oder Methyl,
bedeuten,
zur Herstellung von lichtstabilisiertem EPDM-modifiziertem Polypropylen für den Automobilbau.

2. Verwendung nach Anspruch 1, **dadurch gekennzeichnet, dass** eine Verbindung der Formel (Ia) 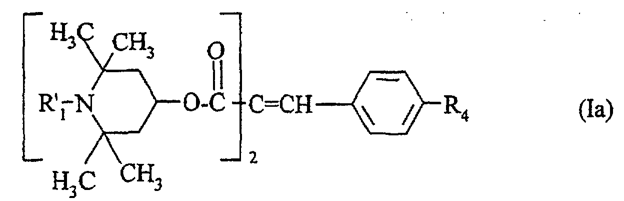 worin
R'₁ = H, CH₃,
R₄ = H, OCH₃
bedeuten,
verwendet wird.

3. Verwendung nach Anspruch 1, **dadurch gekennzeichnet, dass** eine Verbindung der Formel (Ib) 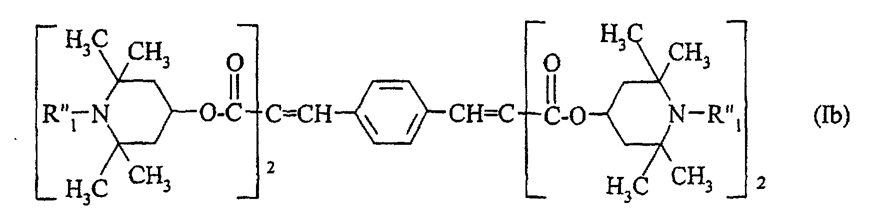 worin
R"₁ = H, CH₃
bedeutet,
verwendet wird.

4. Verwendung nach Anspruch 1, **dadurch gekennzeichnet, dass** eine Mischung aus Piperidinverbindungen der Formel (I) und UV-Absorbern im Verhältnis 10:1 bis 1:10, bevorzugt 4:1 bis 1:4, verwendet wird.

5. EPDM-modifiziertes Polypropylen, das 0,05 bis 2,5 Gew.%, bevorzugt 0,1 bis 0,6 Gew.%, eines Restes einer Verbindung der Formel (I) nach Anspruch 1 enthält.

## Claims

1. Use of piperidine compounds of the formula (I) 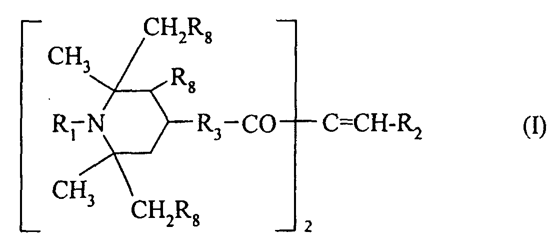 in which
R₁ is hydrogen, acyl or lower alkyl,
R₂ is a substituted or unsubstituted, mono- or bicyclic radical of aromatic type,
R₃ is oxygen, -NH- or -N(C₁₋₄alkyl)-, and
R₈ independently at each occurrence is hydrogen or methyl,
for producing light-stabilized, EPDM-modified polypropylene for automotive construction.

2. Use according to Claim 1, **characterized in that** a compound of the formula (la) 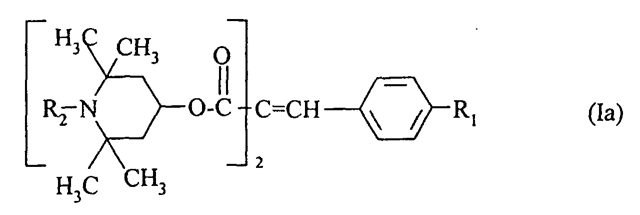 in which R'₁ = H, CH₃; R₄ = H, OCH₃ is used.

3. Use according to Claim 1, **characterized in that** a compound of the formula (Ib) 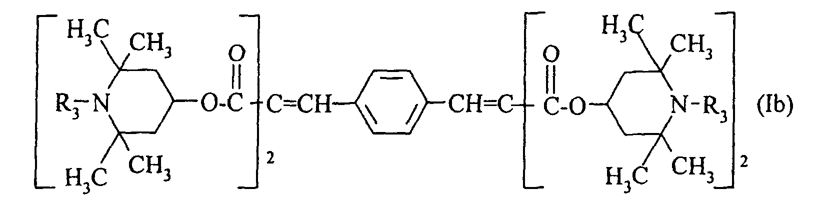 in which R"₁ = H, CH₃ is used.

4. Use according to Claim 1, **characterized in that** a mixture of piperidine compounds of the formula (I) and UV absorbers in a ratio of from 10:1 to 1:10, preferably from 1:4 to 4:1, is used.

5. EPDM-modified polypropylene which contains from 0.05 to 2.5% by weight, preferably from 0.1 to 0.6% by weight, of a radical of a compound of the formula (I) according to Claim 1.

## Revendications

1. Utilisation de composés pipéridiniques de formule (I) 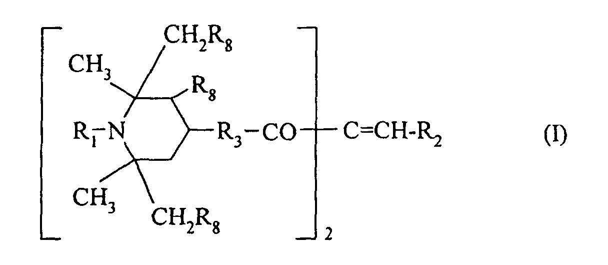 dans laquelle
R₁ signifie l'hydrogène, un groupe acyle ou un groupe alkyle à bas poids moléculaire,
R₂ signifie un reste à caractère aromatique à un ou deux noyaux portant éventuellement des substituants,
R₃ signifie l'oxygène, -NH- ou -N(alkyle en C₁-C₄)- et
tous les symboles R₈ signifient, indépendamment les uns des autres, l'hydrogène ou un groupe méthyle,
pour la préparation de polypropylène modifié EPDM stabilisé contre les effets de la lumière et destiné à la construction d'automobiles.

2. Utilisation selon la revendication 1, **caractérisé en ce qu'**on utilise un composé de formule (la) 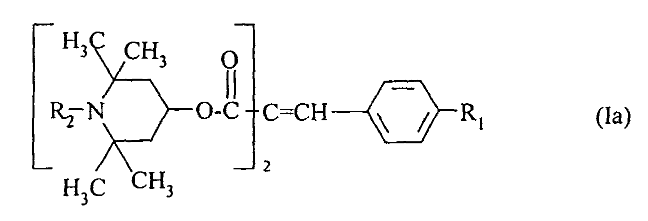 dans laquelle R'₁ = H, CH₃, R₄ = H, OCH₃.

3. Utilisation selon la revendication 1, **caractérisé en ce qu'**on utilise un composé de formule (Ib) 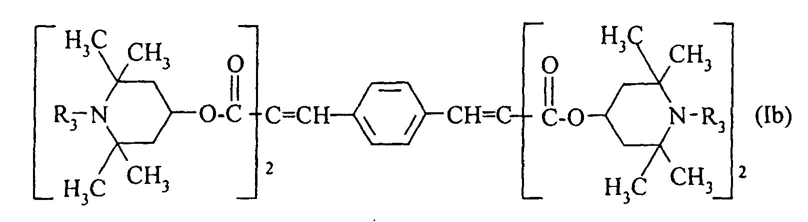 dans laquelle R"₁ = H, CH₃.

4. Utilisation selon la revendication 1, **caractérisé en ce qu'**on utilise un mélange obtenu à partir des composés pipéridiniques de formule (I) et d'absorbants UV dans un rapport de 10:1 à 1:10, de préférence de 4:1 à 1:4.

5. Polypropylène modifié EPDM, qui contient de 0,05 à 2,5% en poids, de préférence de 0,1 à 0,6% en poids, d'un reste d'un composé de formule (I) selon la revendication 1.
